(19) Europäisches Patentamt
European Patent Office
Office européen des brevets

(11) Publication number: 0 319 919 B1

(12) **EUROPEAN PATENT SPECIFICATION**

(45) Date of publication of patent specification: **02.03.94**

(51) Int. Cl.[5]: **C09J 4/06**, C09J 11/06, C08F 20/28, C08G 59/30, //C07D303/24,C07C69/54

(21) Application number: **88120366.5**

(22) Date of filing: **06.12.88**

The file contains technical information submitted after the application was filed and not included in this specification

# (54) Adhesive composition.

(30) Priority: **08.12.87 JP 308556/87**

(43) Date of publication of application: **14.06.89 Bulletin 89/24**

(45) Publication of the grant of the patent: **02.03.94 Bulletin 94/09**

(84) Designated Contracting States: **DE FR GB**

(56) References cited:
**EP-A- 0 212 319**
**EP-A- 0 239 351**
**US-A- 3 879 430**
**US-A- 4 045 408**

**REVIEW OF THE ELECTRICAL COMMUNICATIONS LABORATORIES, vol. 35, no. 3, May 1987, pages 253-257, The Research and Development Headquarters, Tokyo, JP; K. NAKAMURA et al.: "Durable optical adhesive"**

(73) Proprietor: **DAIKIN INDUSTRIES, LIMITED**
**Umeda Center Building,**
**4-12, Nakazaki-nishi 2-chome,**
**Kita-ku**
**Osaka-shi, Osaka-fu(JP)**

Proprietor: **NIPPON TELEGRAPH AND TELEPHONE CORPORATION**
**1-6 Uchisaiwai-cho 1-chome**
**Chiyoda-ku**
**Tokyo(JP)**

(72) Inventor: **Maruno, Tohru**
**3, Higashihara 2-chome**
**Mito-shi Ibaraki-ken(JP)**
Inventor: **Nakamura, Kozaburo**
**12-15, Higashi-koiwa 6-chome**
**Edogawa-ku Tokyo-to(JP)**
Inventor: **Murata, Norio**
**16-2, Sekimachi minami 4-chome**
**Nerima-ku Tokyo-to(JP)**
Inventor: **Kubo, Motonobu**
**13-1, Kumano-cho 4-chome**
**Toyonaka-shi Osaka-fu(JP)**

EP 0 319 919 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

NTIS TECH. NOTES, August 1989, page 677, Springfield, VA, US; "Fluoroepoxy adhesives bond fluoroplastics"

CHEMICAL ABSTRACTS, vol. 88, no. 20, 15th May 1978, pages 20-21, abstract no. 137267d, Columbus, Ohio, US; D.L. HUNSTON et al.: "Fluoro epoxies: surface properties and applications".

Inventor: **Shimizu, Yoshiki**
**72, Ichinoi**
**Akame-cho**
**Nabari-shi Mie-ken(JP)**
Inventor: **Kobayashi, Hideo**
**21-21, Hitotsuya 2-chome**
**Settsu-shi Osaka-fu(JP)**

(74) Representative: **Barz, Peter, Dr. et al**
**Patentanwälte Dipl.-Ing. G. Dannenberg**
**Dr. P. Weinhold, Dr. D. Gudel**
**Dipl.-Ing. S. Schubert, Dr. P. Barz**
**Siegfriedstrasse 8**
**D-80803 München (DE)**

## Description

The present invention relates to a novel adhesive composition comprising a fluorine-containing epoxy resin or fluorine-containing epoxy (meth)acrylate resin.

With a development in optical communication, requirements are increased for organic materials which are suitable to mold a lens or prism or to adhere to an optical part with an another part. Particularly, it is required for an optical adhesive used to adhere to an optical part

(1) to be low in refractive index in order to match with a glass or like optical part in refractive index, and

(2) to be cured at a low temperature in order not to afford a high temperature to the neighboring parts in the adhesion process.

Conventionally, epoxy resin compositions have been employed for that purpose; see e.g. US-A-3 879 430 and 4 045 408 and EP-A-239 351. Since usually available bisphenol A type epoxy resins are too high in refractive index (1.55 to 1.58), an adhesive composition which is low in refractive index and contains a bisphenol A type epoxy resin having introduced fluorine atom therein is disclosed, for example, in Japanese Patent Application No. 217794/1983.

However, these conventional compositions are still high in refractive index (1.51 to 1.58) and have a drawback that the reflection loss is large at the interface with quartz (refractive index 1.46), another optical part. Further, light returning by reflection from the interface lies a defect to render unstable the operation of laser diode light source.

An object of the invention is to provide an adhesive composition which is free from the defect of the conventional epoxy resin having a high refractive index, and is excellent in water-resistance, heat-resistance and adhesive strength, etc.

The present invention relates to an adhesive composition which is characterized by comprising a photopolymerization initiator or curing agent and the compound represented by the formula [I]

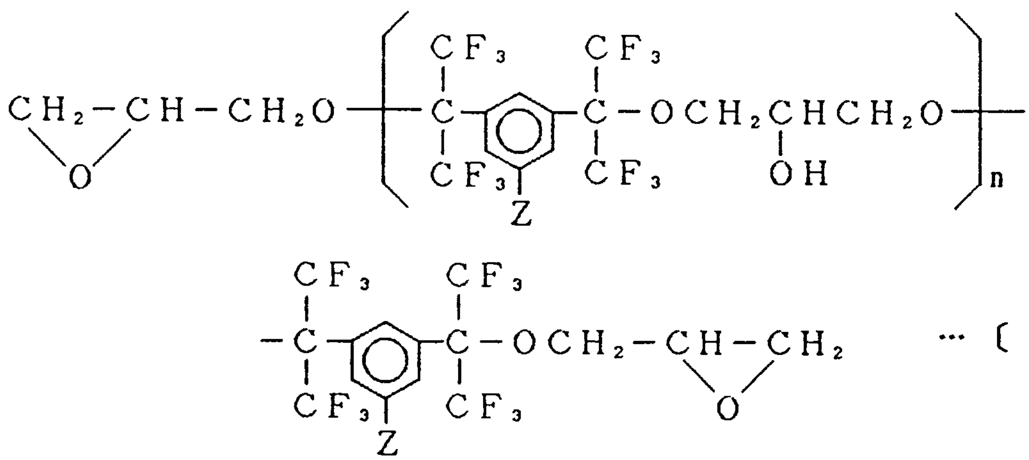

··· [ I ]

wherein n is a number of from 0.1 to 4 and Z is a fluoroalkyl group of 1 to 18 carbon atoms.

The present invention relates to an another adhesive composition which is characterized by comprising a polymerization initiator and the compound represented by the formula [II]

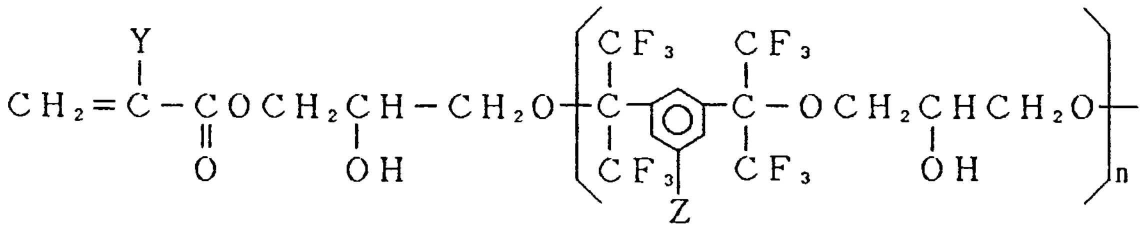

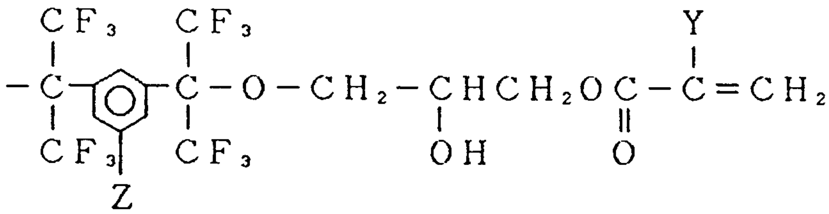

··· [ II ]

wherein n is a number of from 0.1 to 4, Z is a fluoroalkyl group of 1 to 18 carbon atoms, Y is H or $CH_3$.

The present adhesive composition way contain, as other components, for example, conventional epoxy resin, acrylate resins such as epoxy (meth)acrylate resin, urethane acrylate and polybutadiene acrylate and modified products thereof, diluent, curing agent, initiator and coupling agent.

The amount of the fluorine-containing compound of the formula (I) or (II) in the adhesive composition is preferably at least 1% by weight in order to lower the refractive index and enhance water-resistance, heat-resistance and adhesive strength. With less than this amount, the effects are lowered.

Examples of epoxy compounds of the formula (I) of the present invention are compounds having the following structure.

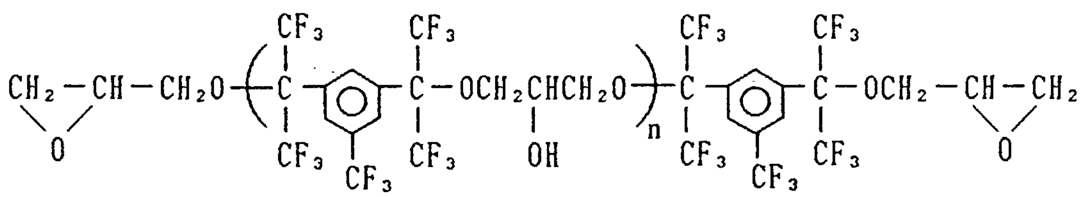

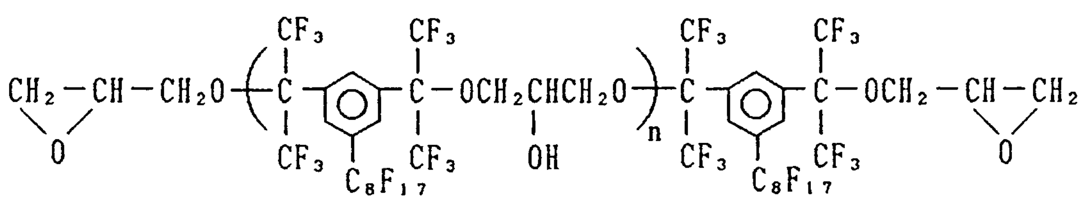

In the above, n is as defined above.

Further, epoxy acrylate compounds of the formula (II) of the present invention include compounds having the following structure and a mixture thereof.

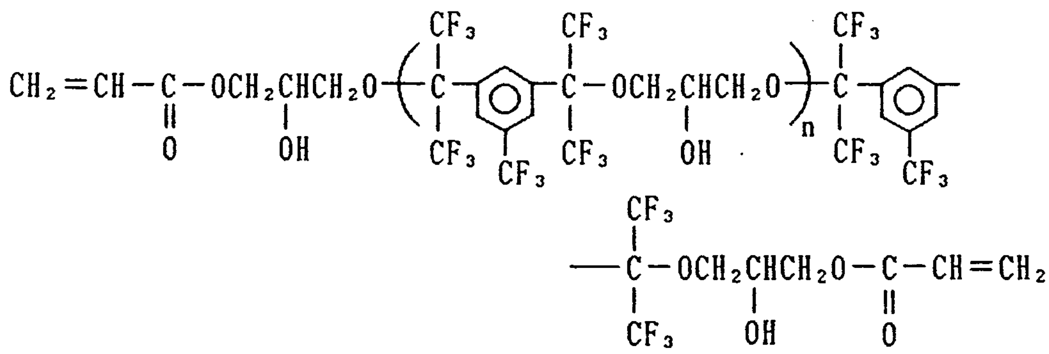

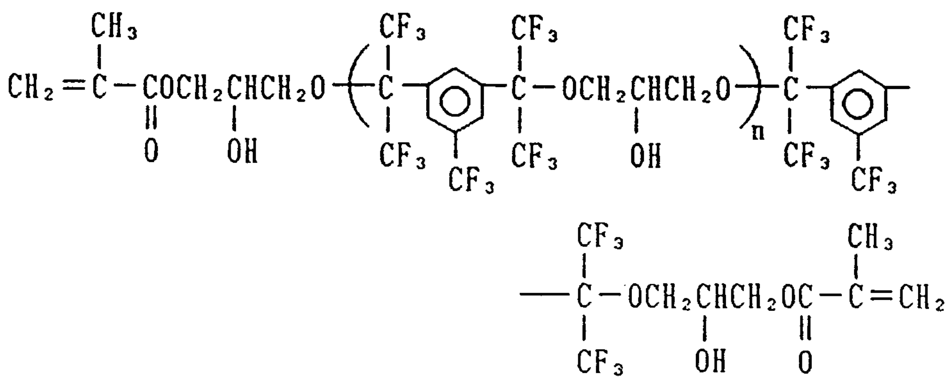

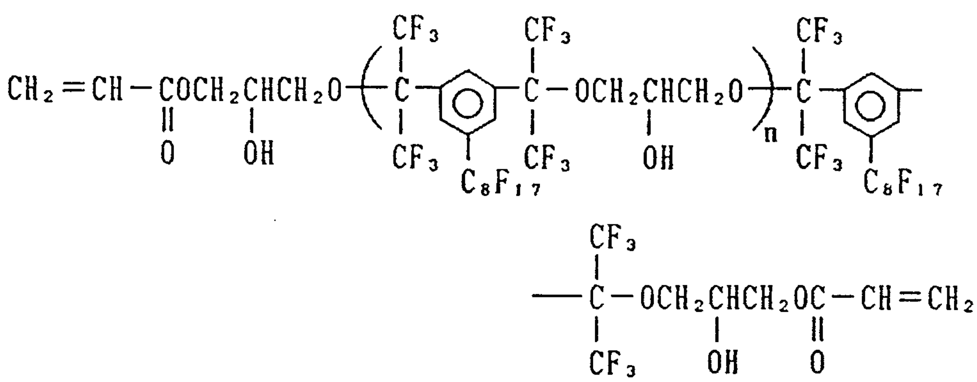

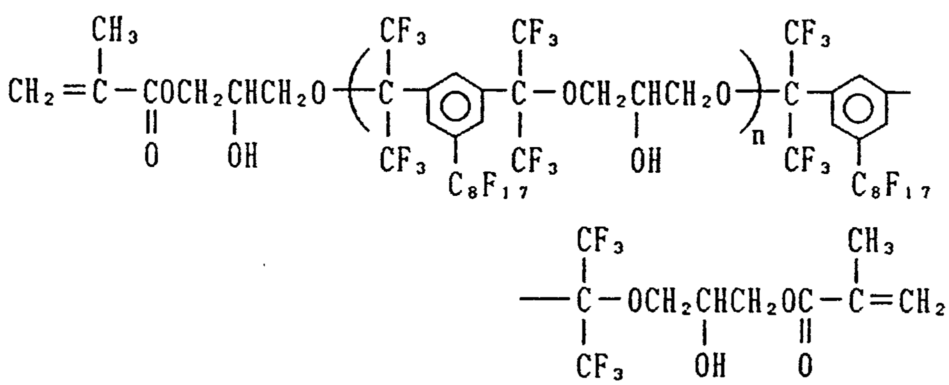

In the above, n is as defined above.

With respect to the epoxy compound of the formula (I) and the epoxy acrylate compound of the formula (II), n is 0.1 to 4 because the compound having a n-value larger than 4 is solid and is difficult to coat.

In the present adhesive composition, examples of epoxy resins conjointly used with the epoxy resin of the formula (I) are those having the following structure, novolak epoxy resin, o-cresol novolak epoxy resin and epoxied polybutadiene.

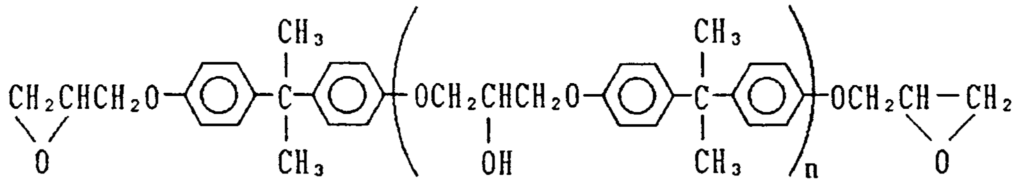

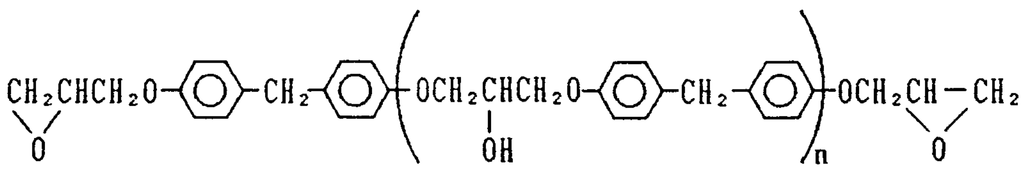

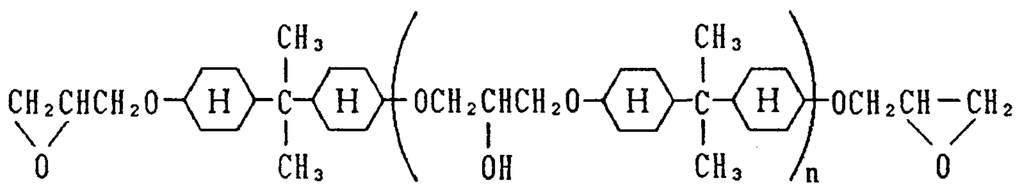

(n is as defined above.)

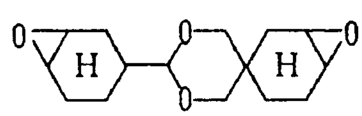

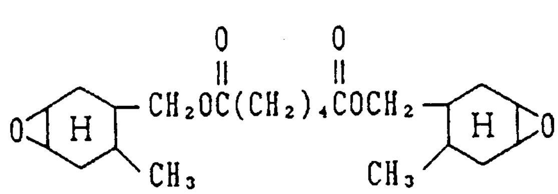

Examples of useful curing agents are polyamines, polyols and acid anhydrides, which are conventionally used for epoxy resins.

Polyamines include diethylenetriamine (DETA), triethylenetetramine (TETA), tetraethylenepentamine (TEPA), dipropylenetriamine (DPTA), bis(hexamethylene)triamine, 1,3,6-trisaminomethylcyclohexane

(TMAH), trimethylhexamethylenediamine (TMD), polyetherpolyamines, diethylaminopropylamine (DEAPA), menthanediamine (MDA), isophoronediamine (IPD), bis(4-amino-3-methylcyclohexyl)-methane, N-aminoethylpiperazine (AEP), m-xylylenediamine (MXDA), m-phenylenediamine (MPDA), diaminodiphenylmethane (DDM), diaminodiphenylsulfone (DDS), 3,9-bis (3 -aminopropyl)-2,4,8,10-tetraspiro[5,5]undecane (ATU) and cyanoethylated polyamines.

In addition, polyamideamines can be used which are prepared mainly by condensation of dimer acid and polyamine. Examples of polyamideamines are Tomide (trade name, same as hereinafter, Fuji chemical Ind. Ltd.), Versamide (Henkel Hakusui Ltd. ), Laquamide (Dainippon Ink and Chemicals, Inc. ), Polymide (Sanyo Chemical Industries Ltd. ), EPOMIK (Mitsui Petrochemical Co., Ltd. ) and Sunmide (Sanwa Chemicals Co., Ltd. ).

Examples of polyols are phenol novolak, o-cresol novolak, polyvinyl phenol, bromide thereof, 2,2-bis(4′-oxyphenyl)propane and 2,2-bis(4′-oxyphenyl)perfluoropropane.

Examples of acid anhydrides are anhydride of each of phthalic acid, trimellitic acid, pyromellitic acid and benzophenonetetracarboxylic acid; anhydride of each of maleic acid, succinic acid, tetrahydrophthalic acid and methyltetrahydrophthalic acid; anhydride of each of methylnadic acid, dodecenylsuccinic acid, hexahydrophthalic acid, methylhexahydrophthalic acid and methylcyclohexenetetracarboxylic acid; anhydride of each of chlorendic acid and tetrabromophthalic acid.

The catalyst for thermal curing includes imidazoles, Lewis acids and the like, which are used for curing the conventional epoxy resins.

Examples of imidazoles are 2-methylimidazole (2MZ), 2-ethyl-4-methylimidazole (2E4MZ), 2-undecylimidazole, 2-heptadecylimidazole, 2-phenylimidazole (2PZ), 1-benzyl-2-methylimidazole (1B2MZ), 1-cyanoethyl-2-methylimidazole (2MZCM), 1-cyanoethyl-2 -ethyl-4-methylimidazole (2E4MZ•CN), 1-cyanoethyl -2-undecylimidazole, 1-cyanoethyl-2-undecylimidazolium• trimellitate, 1-cyanoethyl-2-phenylimidazolium•trimellitate (2PZ•CNS), 2-methylimidazolium• isocyanurate, 2-phenylimidazolium•isocyanurate, 2,4-diamino -6-[2-methylimidazolyl-(1)]-ethyl-s-triazine (2MZ-AZINE), 2,4-diamino-6-[2-ethyl-4-methyl-imidazolyl-(1)]-ethyl-s-triazine (2E4MZ-AZINE) and 2-phenyl-4,5-dihydroxymethylimidazole (2PHZ).

Lewis acids include boron trifluoride ($BF_3$), zinc chloride ($ZnCl_2$), tin tetrachloride ($SnCl_4$), aluminum chloride ($AlCl_3$), phosphorus pentafluoride ($PF_5$), arsenic pentafluoride ($AsF_5$) and antimony pentafluoride ($SbF_5$), and are used usually in the form of an amine-complex.

As a curing catalyst in photopolymerization are used diazonium salts, sulfonium salts, iodonium salts, selenium salts and like compounds, which are known to be effective for epoxy resins.

The diazonium salt is represented by the formula A:

$Ar\text{-}N_2^+X^-$ (A)

Ar includes for example o-, m- or p- nitrophenyl, methoxyphenyl, 2,5-dichlorophenyl, p-(N-morpholino)-phenyl and 2,5-diethoxy-4-(p-trimercapto)phenyl. $X^-$ is an anion and includes $BF_4^-$, $FeCl_4^-$, $PF_6^-$, $AsF_6^-$ and $SbF_6^-$.

As a sulfonium salt are used bis[4-(diphenylsulfonio)phenyl]sulfide-bis-hexafluorophosphate and bis[4-(diphenylsulfonio)phenyl]sulfide-bis-hexafluoroantimonate, and compounds disclosed on page 15, line 24 to page 18, line 1 of Japanese examined patent publication No. 42688/1984.

As a iodonium salt are used di(4-tert-butylphenyl)iodoniumhexafluorophosphateand di(4-tert-butylphenyl)iodoniumhexafluoroantimonate, and compounds disclosed on page 11, line 28 to page 12, line 30 of Japanese examined patent publication No. 42688/1984.

Selenium salts include triphenylseleniumhexafluoroantimonate, 4-tert-butylphenyldiphenyltetrafluoroborate and 2,3-dimethylphenyldiphenylhexafluoroantimonate.

Examples of diluting agents are butyl glycidyl ether, 2-ethylhexyl glycidyl ether and like alkyl ($C_{2\sim25}$) monoglycidyl ether, butanediol diglycidyl ether, 1,6-hexanediol diglycidyl ether, neopentyl glycol diglycidyl ether, dodecanediol diglycidyl ether, pentaerythritol polyglycidyl ether, trimethylolpropane polyglycidyl ether, glycerol polyglycidyl ether, phenyl glycidyl ether, resorcin diglycidyl ether, p-tert-butylphenyl glycidyl ether, allyl glycidyl ether, tetrafluoropropyl glycidyl ether, octafluoropentyl glycidyl ether, dodecafluorooctyl diglycidyl ether, styrene oxide, limonene monoxide, α-pinene epoxide, β-pinene epoxide, cyclohexene epoxide, cyclooctene epoxide, vinylcyclohexene dioxide and the compounds of the following formulae

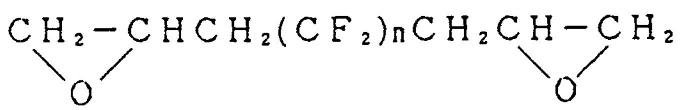

$n = 1 \sim 20$

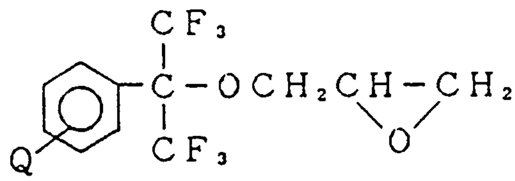

(Q is H, Cℓ, Br, $C_{1\sim18}$ alkyl or $C_{1\sim18}$ fluoroalkyl)

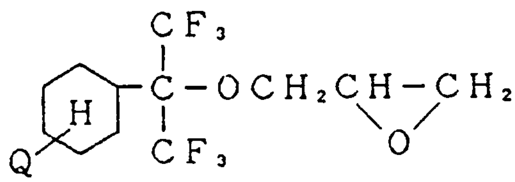

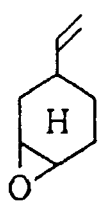 , 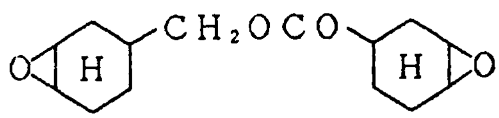

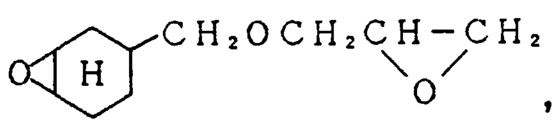 , 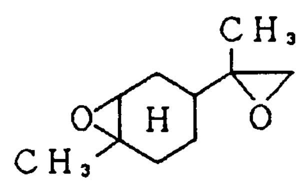

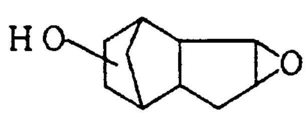 , 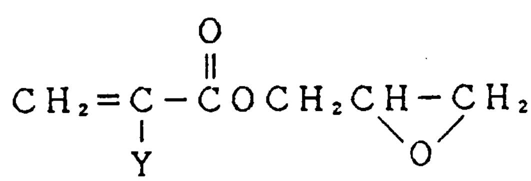

(Y is H or $CH_3$)

As a coupling agent (epoxy type) is used carbon functional silane coupling agent such as γ-mercaptopropyltrimethoxysilane, γ-glycidoxypropyltrimethoxysilane and β-(3,4-expoxycyclohexyl)-ethyltrimethoxysilane.

In the present adhesive composition, the epoxy (meth)acrylate compound of the formula (II) can be mixed with a known reactive oligomer or prepolymer having at least one carbon-carbon double bond in the molecule. Examples threof are shown below.

(1) Ethylenically unsaturated polyesters obtained by condensation oligomerization of a polybasic carboxylic acid, polyol and ethylenically unsaturated monocarboxylic acid

(2) Adducts obtained by the reaction of a polyepoxy compound and ethylenically unsaturated monocarboxylic acid

(3) Esters prepared by the reaction of a polyetherpolyol and ethylenically unsaturated monocarboxylic acid

(4) Ethylenically unsaturated polyurethanes prepared by reacting a polyisocyanate compound and hydroxyalkyl ester of an ethylenically unsaturated monocarboxylic acid

Diallyl phthalate prepolymer, diallyl isophthalate prepolymer and diallyl terephthalate prepolymer can also be used.

More specific example of the polyester (1) is an oligoester (meth)acrylate obtained by condensation oligomerization of maleic anhydride, propylene glycol and (meth)acrylic acid.

More specific examples of the adducts (2) are an epoxy (meth)acrylate obtained from a bisphenol A diglycidyl ether and (meth)acrylic acid, and an epoxy (meth)acrylate obtained from a hydrogenated bisphenol A epoxide and (meth)acrylic acid.

More specific examples of the esters (3) are a polyethylene glycol di(meth)acrylate and polytetraethylene glycol di(meth)acrylate.

More specific example of the polyurethane (4) is a urethane (meth)acrylate, which is obtained by reacting an ethylene glycol and excess of diisocyanate compound to form a urethane prepolymer having isocyanate groups in both terminals and then by reacting therewith 2-hydroxyethyl (meth)acrylate.

Examples of initiators for curing are (a) a thermal polymerization initiator and (b) photopolymerization initiator.

The thermal polymerization initiator includes 2,2′-azobisisobutyronitrile, 2,2′-azobis(2,4-dimethylvaleronitrile), 1-phenylethyl)azodiphenylmethane, 2,2′-azobis(4-methoxy-2,4-dimethylvaleronitrile), dimethyl-2,2′-azobisisobutyrate, 2,2′-azobis(2-methylbutyronitrile), 1,1-azobis(1 -cyclohexanecarbonitrile), 2-(carbomoylazo)-isobutyronitrile, 2,2′-azobis(2,4,4-trimethylpentane), 2-phenylazo-2,4-dimethyl-4-methoxyvaleronitrile, 2,2′-azobis(2-methylpropane) and like azo compounds; benzoyl peroxide, p-chlorobenzoyl peroxide and like diacyl peroxides; methyl ethyl ketone peroxide, cyclohexanone peroxide and like ketone peroxides; tert-butyl perbenzoate, tert-butyl peroxy -2-ethylhexoate and like peresters; tert-butyl hydroperoxide, cumene hydroperoxide and like hydroperoxides; di-tert-butyl peroxide, di-sec-butyl peroxide, dicumyl peroxide and like dialkyl peroxides; and diaryl peroxides.

The photopolymerization initiator includes 2,2-diethoxyacetophenone, 2,2-dimethoxy-2-phenylacetophenone, benzophenone, methyl o-benzoylbenzoate, benzoin isobutyl ether, 2-chlorothioxanthone and 1-(4-isopropylphenyl)-2 -hydroxy-2-methylpropane-1-one.

Examples of reactive diluents are styrene, 1,6-hexanediol di(meth)acrylate, isobornyl (meth)acrylate, diallyl phthalate, diallyl isophthalate, diallyl terephthalate, butylene glycol dimethacrylate, 2-hydroxyethyl methacrylate, 2-hydroxypropyl methacrylate, lauryl acrylate, modified dicyclopentynyl acrylate, glycidyl methacrylate, tetrahydrofurfuryl acrylate, 1,3-butylene glycol dimethacrylate and the compounds of the following formulae having a carbon-carbon double bond

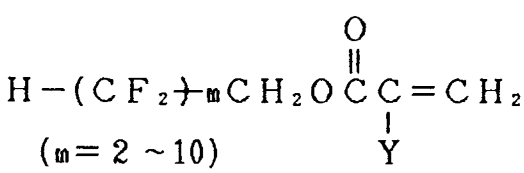

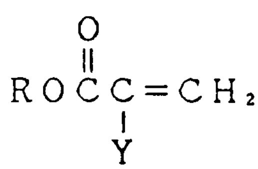

(R is $C_1$ ~ $C_{18}$ alkyl)

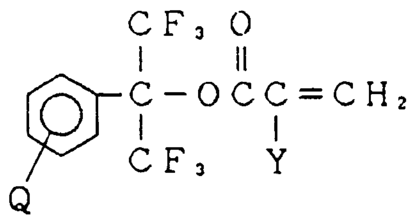

,

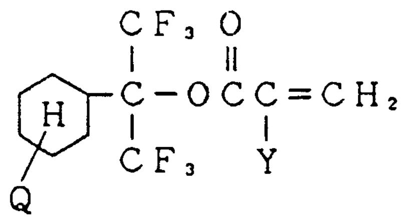

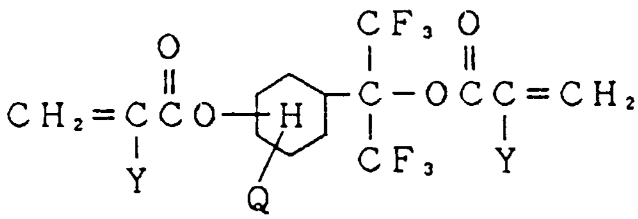

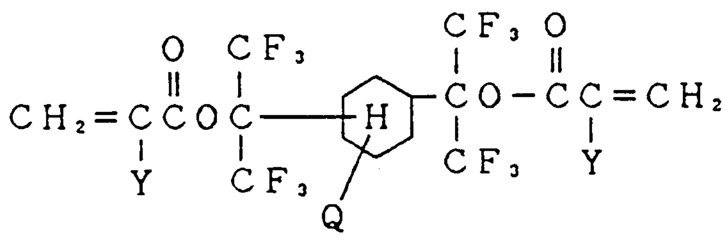

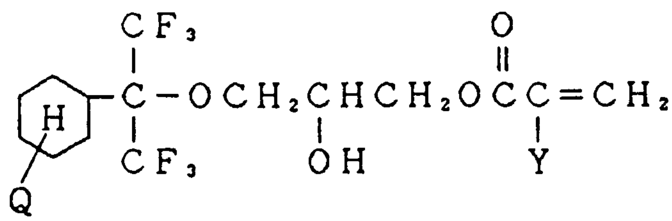

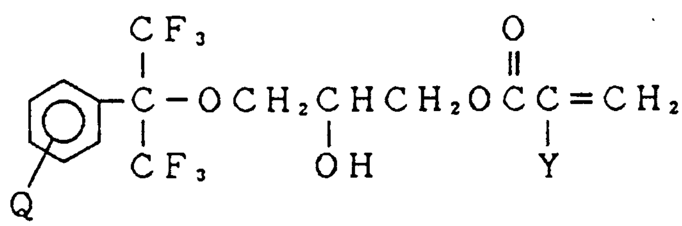

(Q is as defined above)

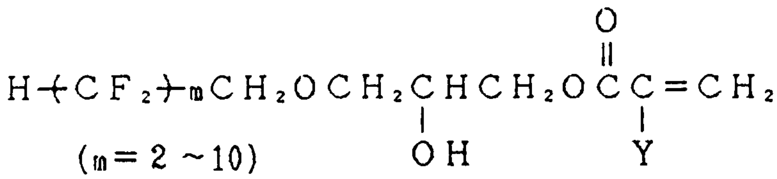

($m = 2 \sim 10$)

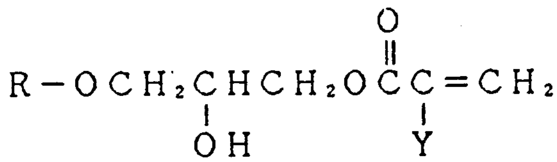

(R is $C_1 \sim C_{18}$ alkyl)

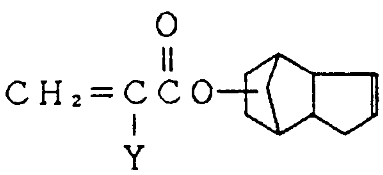

,

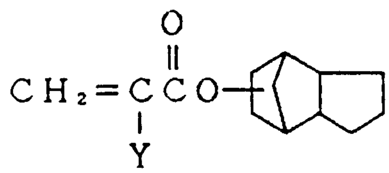

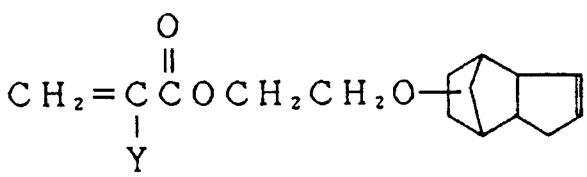

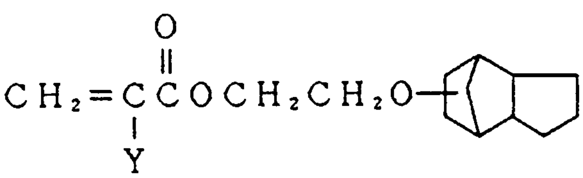

(Y : H or $CH_3$).

Further, it is possible to add trimethoxysilylpropyl (meth)acrylate and like coupling agent to the present adhesive composition.

The present adhesive composition affords a cured product which has a low refractive index and can match with quartz and like optical parts in refractive index, and is excellent in water-resistance, heat-resistance and adhesive strength. Accordingly, the present adhesive composition is useful as an optical adhesive composition for optical parts used in photocommunication system.

The invention will be described in more detail below by showing Reference Examples, Examples and Comparison Examples.

Fig. 1 - 1 is a horizontal sectional view of the test piece for adhesion test made from BK 7 glass plate, Fig. 1 - 2 is a plane schematic view thereof. Fig. 2 - 1 is a horizontal sectinal view of the test piece for heat resistance test made from a glass plate, Fig. 2 - 2 is a plane schematic view thereof. Fig. 3 is a schematic view of a test piece for reflection attenuation test, and Fig. 4 is a graph showing a dependency of the reflection attenuation amount on the refractive index.

1 : BK 7 glass plate, 2,4,7 : adhesive composition and adhesion portion, 3 : slide glass, 5 : optical fiber, 6 : protective tube.

Reference Example 1

To 100mℓ of toluene were added 18.0g (0.25 mole) of acrylic acid and 103g (0.1 mole) of Compound A having the following formula.

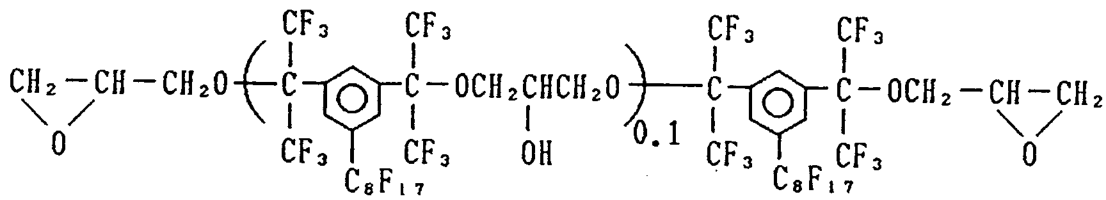

Thereto were added 1 g of trimethylbenzylammonium chloride and 0.1g of p-methoxyphenol and the mixture was heated to reflux with stirring. After 14 hours, the heating was stopped and the mixture was cooled to room temperature. The reaction mixture was washed with a saturated aqueous solution of sodium chloride until the aqueous layer does not indicate acidity and then dried over anhydrous sodium sulfate. Thereto was added 0.1g of p-methoxyphenol and the mixture was heated at 80 to 90°C at a pressure of 1.3 to 2.6 mbar (1 to 2mmHg) to remove the solvent and the compound of the formula [II] wherein n was 0.1, Y was H and Z was $C_8F_{17}$ (Compound B) was obtained in a yield of 98g as a viscous liquid.
IR (liquid film, $cm^{-1}$)
3400 OH, 2900 CH, $CH_2$, 1722 $>C=O$, 1635 $>C=C<$, 1402 $>C=C<$

Reference Example 2

The compound of the formula [II] wherein n was 0.1, Y was $CH_3$ and Z was $C_8F_{17}$ (Compound C) was obtained in a yield of 93g in the same manner as in Reference Example 1 except that 21.5g of methacrylic acid was used in place of acrylic acid.
IR (liquid film, $cm^{-1}$)
3400 OH, 2900 CH, $CH_2$, 1725 $>C=O$, 1640 $>C=C<$, 1404 $>C=C<$

Example 1

To Compound A having the epoxy equivalent of 514 and refractive index of 1.391 was added, as a curing agent, polyether polyamine (PEPA) which is a polyamine type curing agent or boron trifluoride monoethylamine complex (BMA) which is a catalyst for thermal curing, to prepare adhesive compositions. The compositions were cured and checked for refractive index, adhesiveness, glass transition temperature (Tg) and resistance to hot water. The results were given in Table 1.

In the above, the refractive index ($\eta_D^{20}$) was measured by sodium D line at 589.3µm at 20°C with use of Abbé's refractometer. As the adhesiveness, shear adhesive strength was measured at 23°C and at a tensile speed of 5 mm/min. with use of a BK 7 glass test piece shown in Fig.1. Tg was measured by Differential Scanning Calorimetry (DSC). Fig.1-1 is a horizontal sectional view of the test piece for adhesion test made from BK 7 glass plate, Fig. 1 - 2 is a plane schematic view thereof, and 1 is BK 7 glass plate, 2 adhesive composition and adhesion portion. The unit of numerals in Figures is mm.

Heat resistance was checked by measuring a time for separation of a glass test piece shown in Fig. 2 after the immersion in hot water of 80°C. Fig. 2 - 1 is a horizontal sectional view of the test piece for heat resistance test made from a glass plate, Fig. 2 - 2 is a plane schematic view thereof, and 3 is a slide glass, 4 adhesive composition and adhesion portion. The unit of numerals in Figures is mm.

The adhesive composition was cured at 65°C for 6 hours in case of using polyetherpolyamine as a curing agent and at 120°C for 3 hours in case of boron trifluoride monoethylamine.

The followings are an abbreviated name and structure of the diluting agents used.

PRGE : 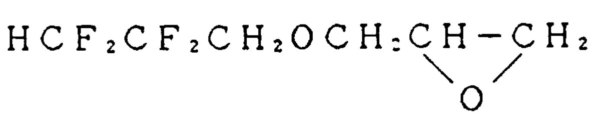

BDDE : 1,4-Butanediol diglycidyl ether

In the below are shown an abbreviated name and structure of the compound used in Comparison Example.

AFEP

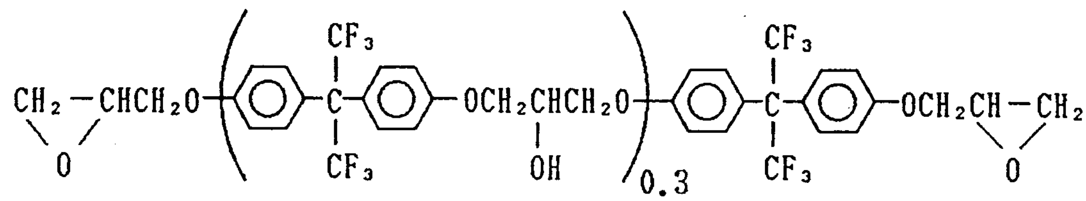

Table 1

| | Epoxy resin name | Epoxy resin part | Diluting agent name | Diluting agent part | Curing agent name | Curing agent part |
|---|---|---|---|---|---|---|
| (a) | Compound A | 100 | PRGE | 20 | PEPA | 30 |
| (b) | Compound A | 100 | PRGE | 20 | BMA | 5 |
| | Comparison | | | | | |
| (c) | AFEP | 80 | BDDE | 20 | PEPA | 40 |

Table 1 (continued)

| | Refractive index | Adhesive strength N/mm² (kgf/cm²) | Tg (℃) | Resistance to hot water (hr) |
|---|---|---|---|---|
| (a) | 1.421 | >17.64 (>180) | 48 | >96 |
| (b) | 1.402 | >18.62 (>190) | 53 | >96 |
| (c) | 1.518 | 10 102 | 76 | 80 |

Example 2

An adhesive composition containing Compound B was cured by irradiation of ultraviolet ray of 100 mJ/cm². The cured product was checked in the same manner as in Example 1 in the refractive index, adhesive strength, Tg and resistance to hot water. In the below are shown an abbreviated name and structure of the compound used for comparison.

AFEPA

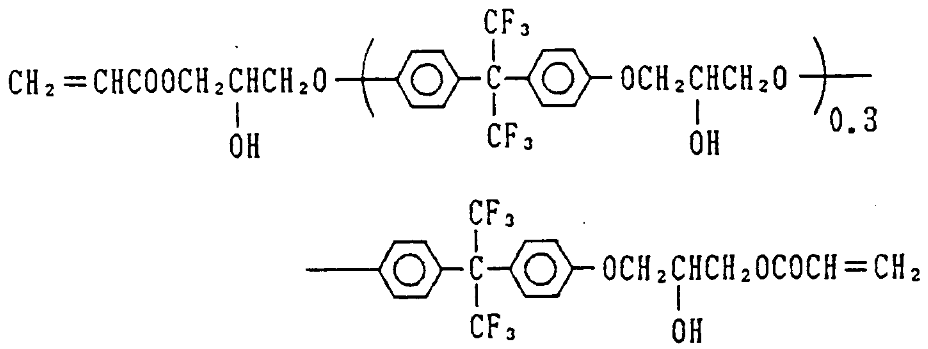

Shown below is an abbreviated name and structure of the diluting agent used.

EGDMA :

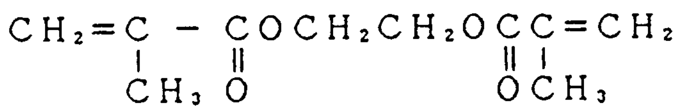

As a photopolymerization initiator was used benzoin isobutyl ether (BBE). The results were given in Table 2.

Table 2

| | Resin | | Diluting agent | | Photopolym. initiator | |
|---|---|---|---|---|---|---|
| | name | part | name | part | name | part |
| (a) | Compound B | 100 | EGDMA | 20 | BBE | 3 |
| | Comparison | | | | | |
| (b) | AFEPA | 100 | EGDMA | 20 | BBE | 3 |

Table 2 (continued)

| | Refractive index | Adhesive strength N/mm² (kgf/cm²) | Tg (℃) | Resistance to hot water (hr) |
|---|---|---|---|---|
| (a) | 1.418 | 10.39 (106) | 62 | >24 |
| (b) | 1.528 | 4.9 (50) | 98 | >24 |

## Claims

**1.** An adhesive composition comprising a photopolymerization initiator or curing agent and the compound represented by the formula [I]

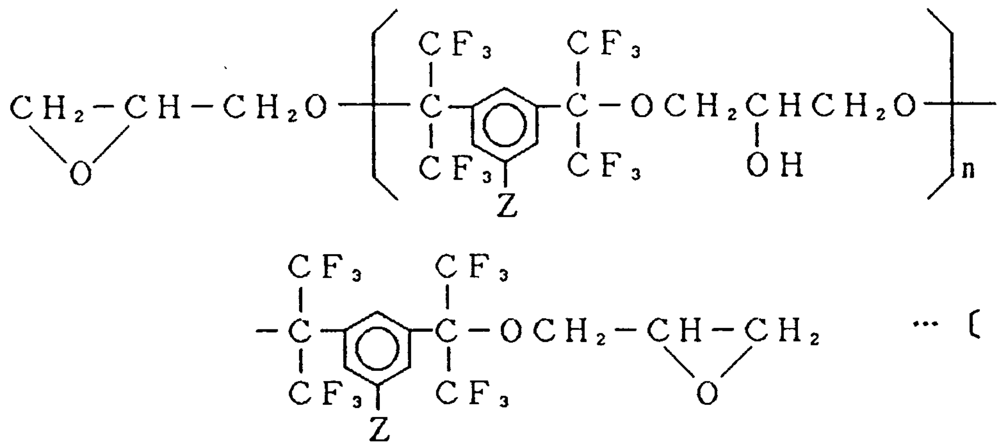

··· [ I ]

wherein n is a number of from 0.1 to 4 and Z is a fluoroalkyl group of 1 to 18 carbon atoms.

**2.** An adhesive composition comprising a polymerization initiator and the compound represented by the formula [II]

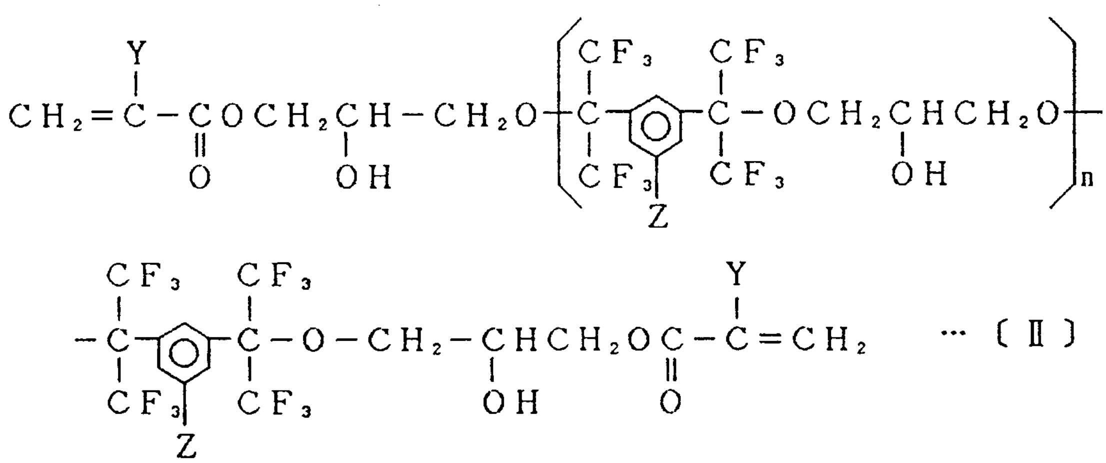

··· [ II ]

wherein n is a number of from 0.1 to 4, Z is a fluoroalkyl group of 1 to 18 carbon atoms, Y is H or $CH_3$.

## Patentansprüche

**1.** Klebstoff-Zusammensetzung, umfassend einen Photopolymerisationsinitiator oder Härter und eine Verbindung der Formel (I)

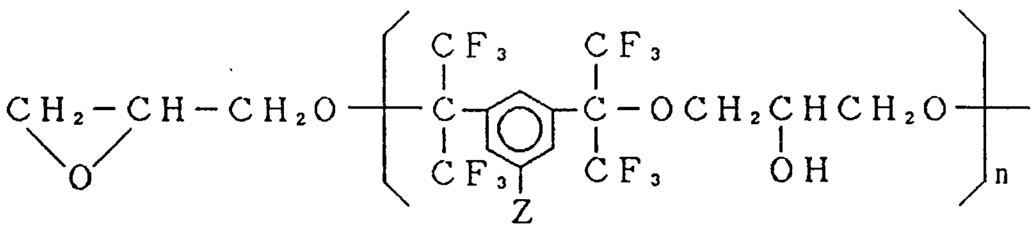

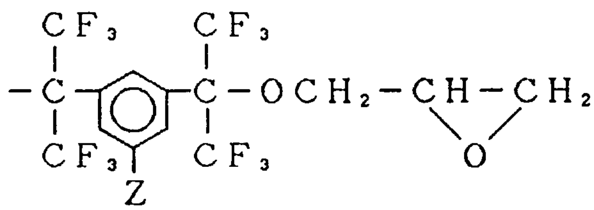

··· [ I ]

worin n ein Zahl von 0,1 bis 4 ist und Z eine Fluoralkylgruppe mit 1 bis 18 Kohlenstoffatomen ist.

**2.** Klebstoff-Zusammensetzung, umfassend einen Polymerisationsinitiator und eine Verbindung der Formel (II)

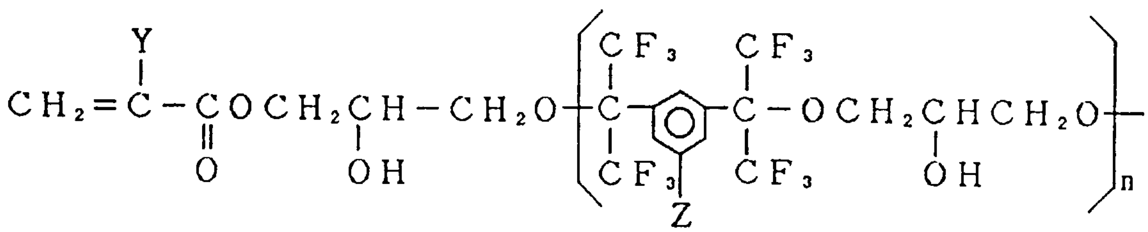

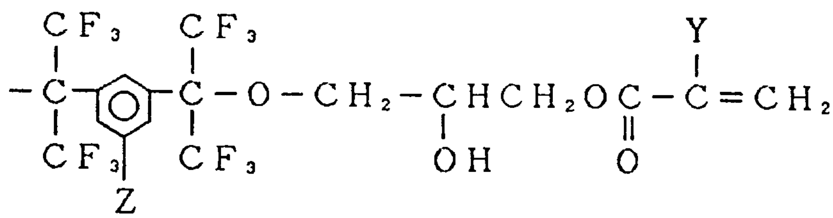

··· [ II ]

worin n eine Zahl von 0,1 bis 4 ist, Z eine Fluoralkylgruppe mit 1 bis 18 Kohlenstoffatomen ist und Y H oder $CH_3$ ist.

**Revendications**

**1.** Composition adhésive comprenant un initiateur de photopolymérisation ou un agent de durcissement et le composé représenté par la formule (I) :

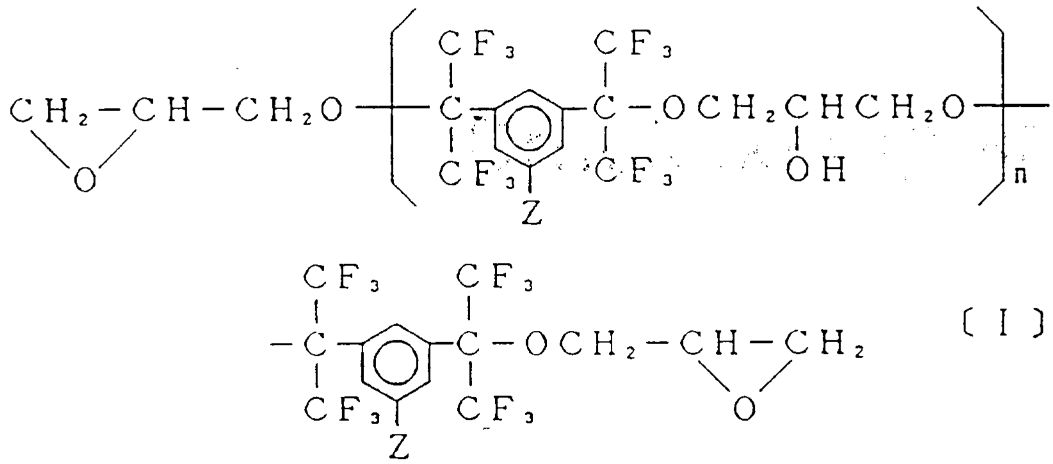

( I )

dans laquelle n est un nombre de 0,1 à 4 et Z est un groupe fluoroalkyle de 1 à 18 atomes de carbone.

2. Composition adhésive comprenant un initiateur de polymérisation et le composé représenté par la formule (II) :

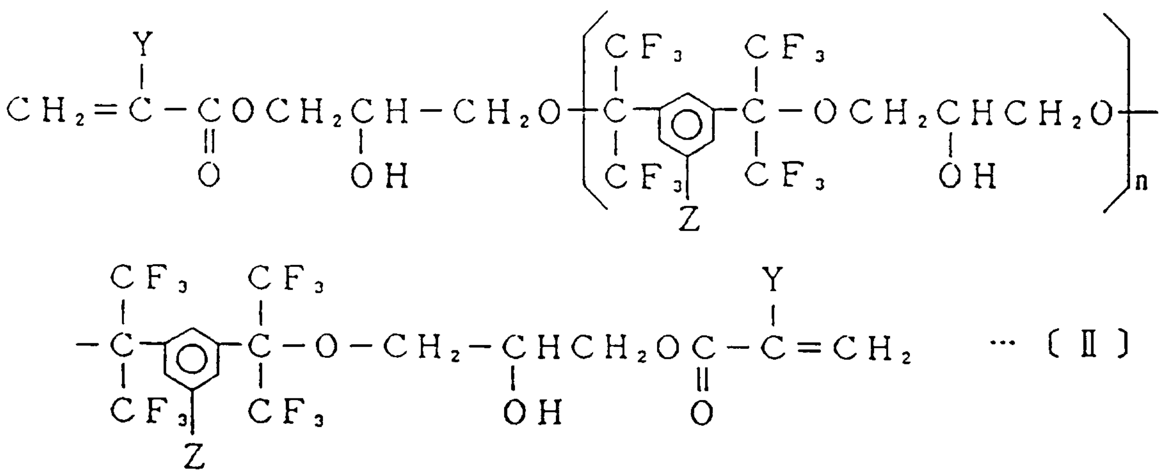

··· ( II )

dans laquelle n est un nombre de 0,1 à 4 et Z est un groupe fluoroalkyle de 1 à 18 atomes de carbone, Y est un atome d'hydrogène ou un groupe $CH_3$.

Fig. 1-1 Fig. 1-2

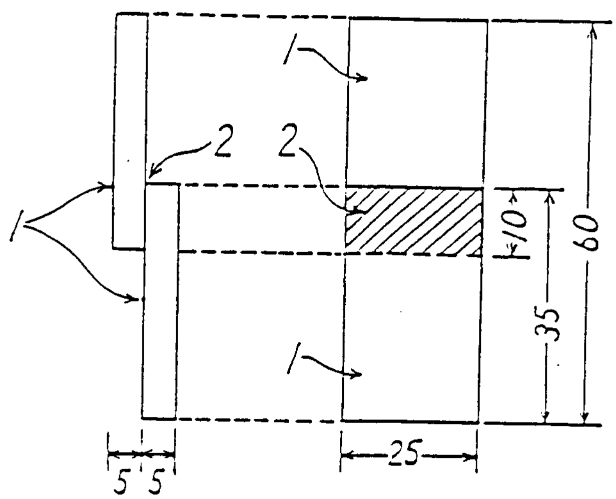


Fig. 2-1

Fig. 2-2

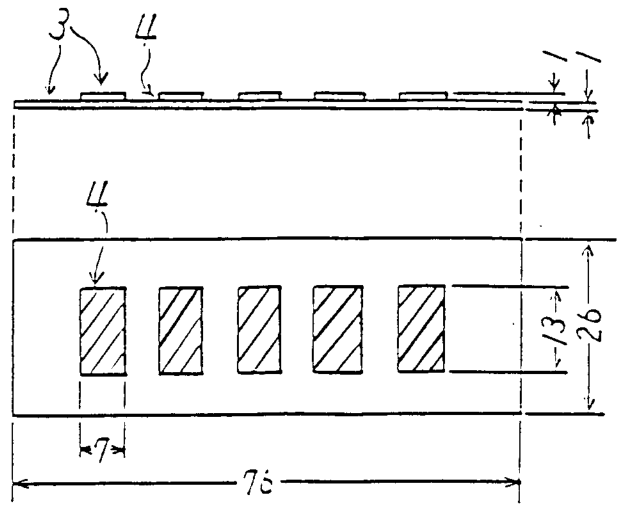

Fig. 3

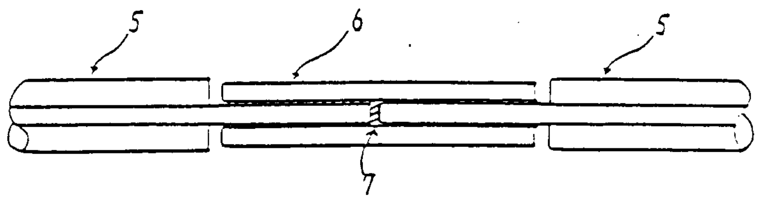
5
6
5
7

Fig. 4

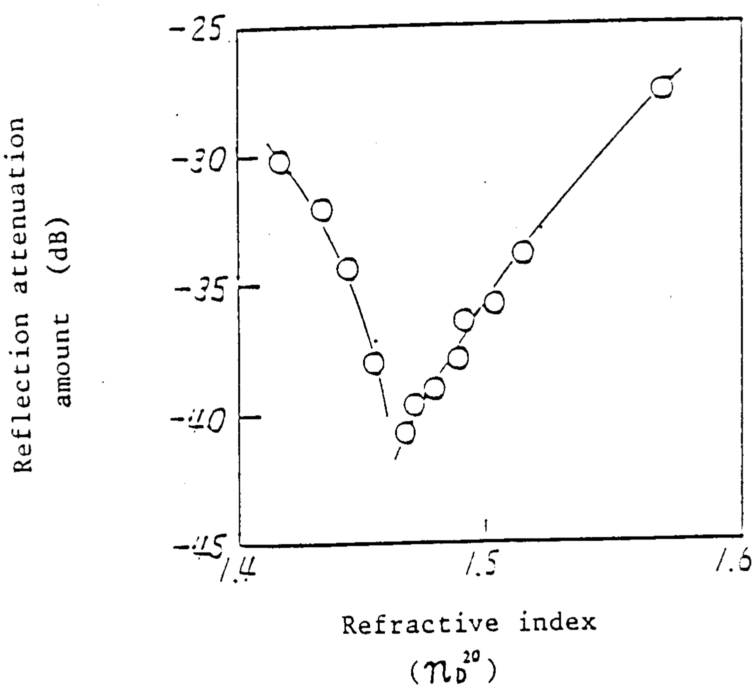
-25
-30
-35
-40
-45
1.4
1.5
1.6
Reflection attenuation amount (dB)
Refractive index
($n_D^{20}$)